(19) Europäisches Patentamt
European Patent Office
Office européen des brevets

(11) **EP 2 324 769 A1**

(12) **EUROPEAN PATENT APPLICATION**
published in accordance with Art. 153(4) EPC

(43) Date of publication:
**25.05.2011 Bulletin 2011/21**

(51) Int Cl.:
***A61B 8/00*** *(2006.01)*

(21) Application number: **09812985.1**

(22) Date of filing: **20.08.2009**

(86) International application number:
**PCT/JP2009/064576**

(87) International publication number:
**WO 2010/029838 (18.03.2010 Gazette 2010/11)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **09.09.2008 JP 2008230428**

(71) Applicant: **Hitachi Medical Corporation**
**Chiyoda-ku**
**Tokyo 101-0021 (JP)**

(72) Inventor: **FUJII,Nobuhiko**
**Tokyo 101-0021 (JP)**

(74) Representative: **MERH-IP**
**Matias Erny Reichl Hoffmann**
**Paul-Heyse-Strasse 29**
**80336 München (DE)**

(54) **ULTRASONOGRAPHIC DEVICE, ULTRASONOGRAPHIC DEVICE DATA PROCESSING METHOD**

(57)    An ultrasonic diagnostic apparatus having an ultrasonic probe for transmitting an ultrasonic beam to an examinee and receiving a reflection echo signal from the examinee, a beam forming unit for supplying the ultrasonic probe with a signal for making the ultrasonic probe transmit an ultrasonic beam, and a data converter for digitalizing the reflection echo signal to obtain echo data, and converting the echo data to ultrasonic image data including a setting unit that sets measurement mode data of the ultrasonic image data in the echo data, and a controller that creates a data set while appending the measurement mode data and the echo data, transferring the data set to the data converter, makes the data converter analyze the measurement mode data in the data set, and controls the data converter so that echo data in the data set are converted to ultrasonic image data on the basis of the analyzed measurement mode data.

FIG. 1

# Description

Technical Field

The present invention relates to an ultrasonic diagnostic apparatus and a data processing method for the same, and particularly to a technique of an image operating device for converting a reflection echo signal to an ultrasonic image.

Background Art

A conventional ultrasonic diagnostic apparatus has a function of measuring a tomogram (B mode image) of an examinee.
Furthermore, for example when a tomogram of a heart is measured, the ultrasonic diagnostic apparatus has a function of measuring a time-varying image (M mode image) of the wall of the heart. In order to measure these plural measurement modes in parallel, plural data converters called as a digital scan converter (DSC) are provided (for example, Patent Document 1).

Prior Art Document

Patent Document

Patent Document 1: Japanese Patent No. 3,612,358

Summary of the Invention

Problem to be Solved by the Invention

However, the ultrasonic diagnostic apparatus still has a technical problem that tradeoff occurs between reduction of the circuit scale of a data converter which contributes to miniaturization of the device and speed-up of data operation processing of the data converter which contributes to enhancement of the function of the device.
Therefore, an object of the present invention is to provide an ultrasonic diagnostic apparatus that can speed up data operation processing even when only one data converter is provided, and a data processing method for the ultrasonic diagnostic apparatus.

Means of solving the Problem

In order to solve the foregoing problem, the present invention is constructed as follows.
An ultrasonic diagnostic apparatus according to the present invention that has an ultrasonic probe for transmitting an ultrasonic beam to an examinee and receiving a reflection echo signal from the examinee, a beam forming unit for supplying the ultrasonic probe with a signal for making the ultrasonic probe transmit an ultrasonic beam, and a data converter for digitalizing the reflection echo signal to obtain echo data, and converting the echo data to ultrasonic image data, includes a setting unit that sets measurement mode data of the ultrasonic image data in the echo data, and a controller that creates a data set while appending the measurement mode data and the echo data, transferring the data set to the data converter, makes the data converter analyze the measurement mode data in the data set, and controls the data converter so that echo data in the data set are converted to ultrasonic image data on the basis of the analyzed measurement mode data.
Furthermore, a data processing method for an ultrasonic diagnostic apparatus according to the present invention includes: transmitting an ultrasonic beam to an examinee and receiving a reflection echo signal from the examinee by an ultrasonic probe; supplying the ultrasonic probe with a signal for making the ultrasonic probe transmit an ultrasonic beam by a beam forming unit; digitalizing the reflection echo signal to obtain echo data and converting the echo data to ultrasonic image data by a data converter; setting measurement mode data of the ultrasonic image data in the echo data by a setting unit; and generating data set while appending the measurement mode data and the echo data, transferring the data set to the data converter, making the data converter analyze measurement mode data in the data set, and controlling the data converter so as to convert echo data in the data set to ultrasonic image data on the basis of the analyzed measurement mode data by a controller.
These constituents can speed up the data operation processing even by one data converter.

Effect of the Invention

As described above, according to the present invention, there can be provided an ultrasonic diagnostic apparatus that can speed up the data operation processing even by one data converter, and a data processing method for the ultrasonic diagnostic apparatus.

Brief Description of the Drawings

[Fig. 1] Fig. 1 is a block diagram showing the construction of an ultrasonic diagnostic apparatus to which the present invention is applied.
[Fig. 2A] Fig. 2A is a diagram showing a construction of generating a data set of shared data and RF data and a data flow.
[Fig. 2B] Fig. 2B is a diagram showing a construction of generating a data set of shared data and RF data and a data flow.
[Fig. 3] Fig. 3 is a diagram showing a construction of generating packet data from original data and a data flow.
[Fig. 4] Fig. 4 is a diagram showing a construction of restoring original data from packet data and a data flow.

[Fig. 5] Fig. 5 is a block diagram showing an example in which the construction of the ultrasonic diagnostic apparatus of Fig. 1 is made to process a B mode image.

[Fig. 6] Fig. 6 is a block diagram showing an example in which the construction of the ultrasonic diagnostic apparatus of Fig. 1 is made to process a B mode image.

[Fig. 7] Fig. 7 is a time chart showing an executing cycle of data processing of BM1, BM2 of Fig. 5 and a processing flow of assigning processing to BM1, BM2 of Fig. 5 to perform specific processing of generating the B mode image by a pipeline system.

[Fig. 8] Fig. 8 is a block diagram showing an example in which the construction of the ultrasonic diagnostic apparatus of Fig. 1 is made to perform processing of DEMUX processing, B mode, D mode measurement.

[Fig. 9] Fig. 9 is a processing flow of assigning processing to DEM, BM1, BM2, DM1, DM2 of Fig. 8 to perform specific processing of imaging the B mode image and the D mode measurement by a pipeline system.

[Fig. 10] Fig. 10 is a time chart showing an executing cycle of data processing of DEM, BM1, BM2, DM1, DM2 of Fig. 8.

[Fig. 11] Fig. 11 is a block diagram showing an example in which the construction of the ultrasonic diagnostic apparatus of Fig. 1 is made to perform B mode (containing the DEMUX processing), D mode measurement (containing DEMUX processing).

[Fig. 12] Fig. 12 is a diagram showing a processing flow of assigning processing to BM1, BM2, DM1, DM2 of Fig. 11 to perform specific processing of imaging the B mode image and the D mode measurement by the pipeline system.

[Fig. 13] Fig. 13 is a time chart showing an executing cycle of data processing of BM1, BM2, DM1, DM2 of Fig. 11.

[Fig. 14] Fig. 14 shows a processing flow of interrupting a pipeline of a specific processing of imaging the B mode image.

[Fig. 15] Fig. 15 is a time chart showing an executing cycle of data processing of BM1 of Fig. 14.

Embodiments for carrying out the Invention

An ultrasonic diagnostic apparatus to which the present invention is applied will be described with reference to the drawings.

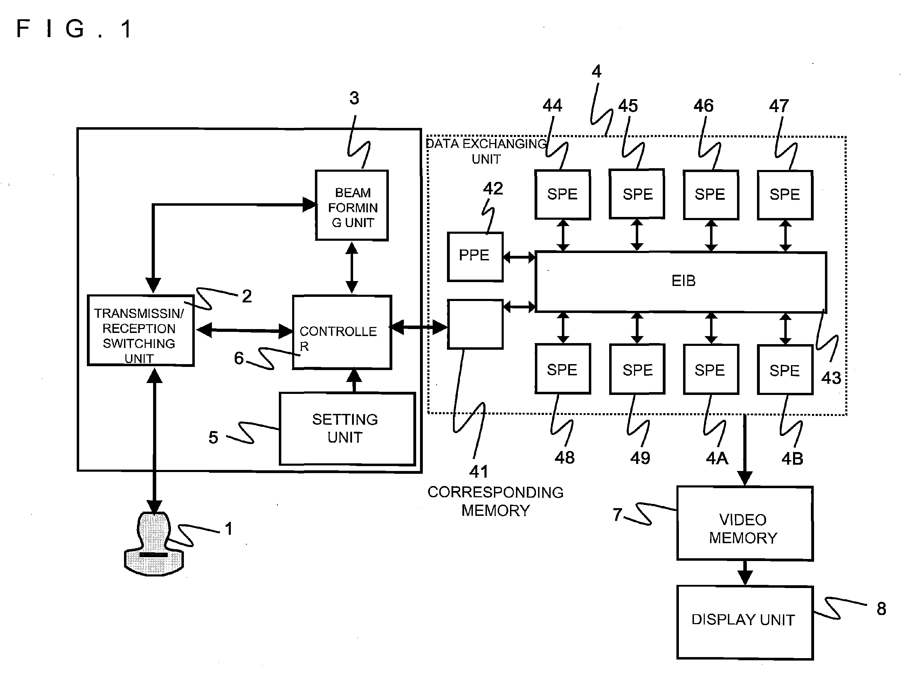

Fig. 1 is a block diagram showing the construction of the ultrasonic diagnostic apparatus to which the present invention is applied.

As shown in Fig. 1, the ultrasonic diagnostic apparatus according to the present invention has an ultrasonic probe 1 for transmitting an ultrasonic beam to an examinee and receiving a reflection echo signal from the examinee, a transmission/reception switching unit 2 for switching transmission and reception of the ultrasonic probe 1, a beam forming unit (DBF: Digital Beam Former) 3 for supplying the ultrasonic probe 1 with a signal which makes the ultrasonic probe 1 transmit an ultrasonic beam, a data converter (DSC: Digital Scan Converter) 4 for digitalizing the reflection echo signal to obtain echo data and converting the echo data to ultrasonic image data, a setting unit 5 for setting measurement mode data of the ultrasonic image data in the echo data, and a controller (Controller) 6 for generating a data set for the measurement mode data and the echo data, transferring the data set to the data converter 4, making the data converter 4 analyze the measurement mode data in the data set, and controlling the data converter 4 to convert the echo data in the data set to ultrasonic image data on the basis of the analyzed measurement mode data.

In the ultrasonic probe 1, transducer elements which correspond to 1 to m channels are arranged in the long axis direction of the ultrasonic probe 1. In this case, the ultrasonic probe 1 is cut into m parts in the long axis direction in connection with 1 to m channels. Therefore, beam focus of wave transmission and wave reception can be performed in the long axis direction by changing a delay time applied to each of the transducer elements (1 to m channels) in the long axis direction. Furthermore, transmission-wave weighting is performed by varying the amplitude of an ultrasonic transmission signal to be supplied to each transducer element in the long axis direction, and reception-wave weighting is performed by varying the amplification degree or attenuation degree of an ultrasonic reception signal from each transducer element in the longitudinal direction. Furthermore, aperture control can be performed by turning on/off each transducer element in the short axis direction. Here, when the ultrasonic probe 1 is also cut into k parts in the short axis direction so that the k parts are arranged in connection with 1 to k channels, beam focus of transmission wave and reception wave can be also performed in the short axis direction by changing the delay time applied to each transducer element (1 to k channels) in the short axis direction.

Furthermore, transmission-wave weighting is performed by varying the amplitude of the ultrasonic transmission signal applied to each transducer element in the short axis direction, and reception-wave weighting is performed by varying the amplification degree or attenuation degree of the ultrasonic reception signal from each transducer element in the short axis direction. Furthermore, aperture control can be performed by turning on/off each transducer element in the short axis direction.

There is also known the ultrasonic probe 1 in which a transducer is formed of a piezoelectric element. Furthermore, there is also known the ultrasonic probe 1 in which a transducer is formed of a semiconductor called as CMUT (Capacitive Micro machined Ultrasonic Transducer).

The transmission/reception switching unit 2 serves as an interface for supplying a transmission signal to the ultrasonic probe 1 and also processing a received reflection echo signal. Furthermore, the transmission/reception switching unit 2 has a function of a wave receiving circuit for receiving the reflection echo signal from the inside of the examinee based on the punch-out ultrasonic beam to collect biological information.

The beam forming unit 3 is a wave transmission circuit for controlling the ultrasonic probe 1 to punch out an ultrasonic beam.

The data converter 4 converts the reflection echo signal processed in the transmission/reception switching unit 2 to an ultrasonic tomographic image, and forms an ultrasonic image on the basis of successively input reflection echo signals. The ultrasonic image contains an A mode image, a B mode image, a color flow mapping (C) mode image, a Doppler (D) mode image, an elasticity (elast) (E) mode image, and a three-dimensional ultrasonic image obtained by mainly reconstructing B mode images which are arranged continuously along the body surface of the examinee.

The setting unit 5 is used for an operator to input various kinds of desired parameters such as a measurement mode, patient information, an imaging position, etc. by using a key board or a trackball.

The controller 6 is a control computer system for controlling the transmission/reception switching unit 2, the beam forming unit 3 and the data converter 4 to function on the basis of the various kinds of parameters input by the setting unit 5.

A video memory 7 stores an ultrasonic image formed by the data converter 4, character and graphic information such as patient information and body mark information and graphic information of the setting unit 5 while combining these information. The controller 6 described above also has the function of a display controller for selectively controlling a display format for display.

A display unit 8 displays an ultrasonic image stored in the video memory 7, and it includes a CRT monitor or liquid crystal monitor, for example. The display unit 8 may be configured to display an ultrasonic image so that the operator can make diagnoses, and any display technique of analog output and digital output may be contained in the embodiment of this invention.

Furthermore, the data converter 4 has an internal memory 41, a processor controller (PPE: PowerPC Processor Element) 42, an internal data transmission bus (EIB: Element Interconnect Bus) 43, and processors (SPE: Synergistic Processor Element) 44 to 4B.

The internal memory 41 receives and stores data transmitted from the controller 6.

The processor controller 42 controls the processors 44 to 4B which are connected to one another through the internal data transmission bus 43. As a specific example of the control thereof, it analyzes data and parameters stored in the internal memory 41, assigns processing programs to the processors 44 to 4B and obtain an ultrasonic image from the data stored in the internal memory 41. A processor in which architectures such as CELL, etc. are standardized is used as the data converter 4. CELL is an abbreviation of Cell Broadband Engine (registered trademark) in Japan, and it is a microprocessor developed by Sony Computer Entertainment Co., Ltd., etc.

Furthermore, the number of the processors is set to eight as an example in Fig. 1, however, it may be set to any natural number because any number can be set in accordance with the processing capability of the processor.

(First Embodiment) Data set of shared data (CD) and processing target data (RD)/transmission

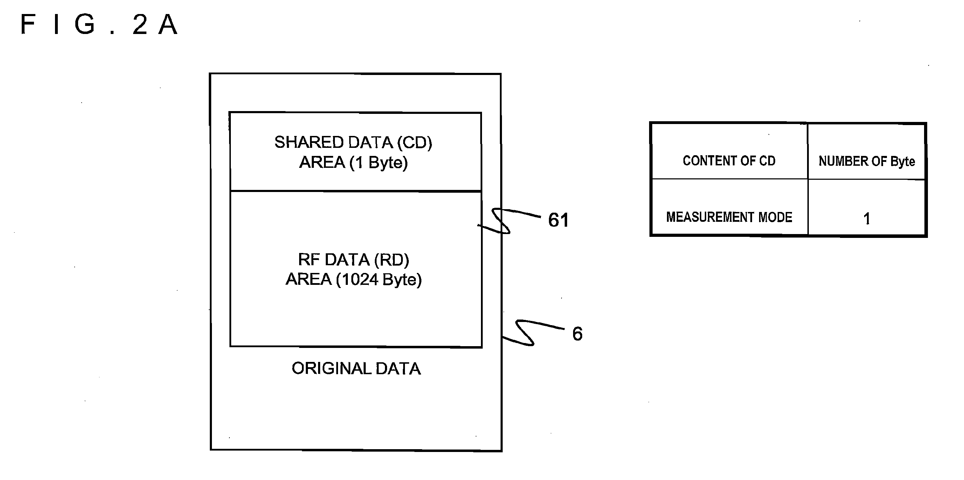

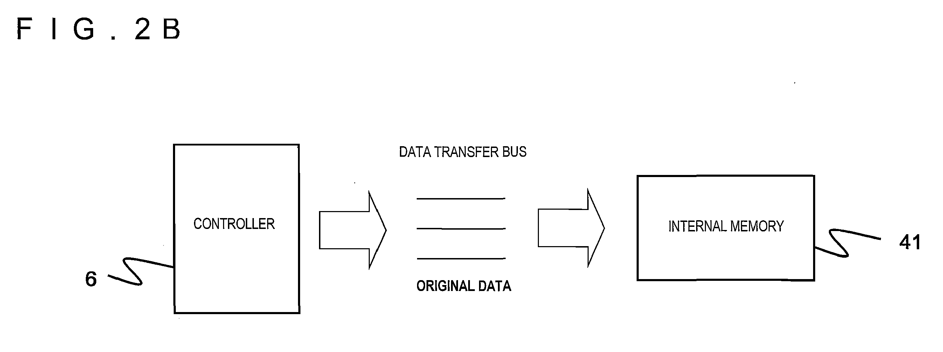

A first embodiment will be described with reference to Figs. 2A and 2B. In the fist embodiment, the data set of the shared data (CD) and the processing target data (RD) and a data processing method based on this data set will be disclosed. The data set means that composite data of the shared data (CD) and the processing target data (RD) are created.

Figs. 2A and 2B are diagrams showing a construction of generating the data set of the shared data and the RF data and a data flow.

First, the construction of the first embodiment will be described with reference to Fig. 2A.

The controller 6 has an internal memory 61.

The internal memory 61 stores the shared data (CD) containing measurement mode (A mode, B mode, C mode, D mode, E mode, three-dimension, etc.) data set by the setting unit 5. The shared data (CD) has a storage area of 1 byte as an example.

The internal memory 61 stores the measured processing target data (RD). The processing target data (RD) has a storage area of 1024 bytes as an example.

The controller 6 has a function of consecutively storing addresses of the storage area of the shared data (CD) and the storage area of the processing target data (RD) in the internal memory 61. In this embodiment, this function is referred to as ""data set" established". The controller 6 also has a function of transmitting to the memory 41 of the data converter 4 (DSC) the data stored in the storage areas of the shared data (CD) and the processing target data (RD) for which data set is established.

The operation of the first embodiment will be described with reference to Fig. 2B.

The controller 6 makes the address of the storage area of the shared data (CD) and the address of the storage area of the processing target data (RD) in the internal memory 61 consecutive to establish the data set.

The controller 6 transmits to the memory 41 of the data converter 4 the data stored in the storage areas of the shared data (CD) and the processing target data (RD) for which the data set is established.

According to the first embodiment, the controller 6 generates the data set for the shared data (CD) and the processing target data (RD) and transmits the data set to the memory 41 of the data converter 4, makes the data converter 4 analyze the measurement mode data in the data set and controls the data converter 4 with the analyzed measurement mode data to convert the echo data in the data set to the ultrasonic image data. Therefore, the processing speed of the data operation processing can be increased by even one data converter. Furthermore, an effect inherent to the first embodiment resides in that the processing target data and the processing parameters are delivered to the processor together through the data transmission bus, and thus standby processing which has been hitherto provided when arrival of a signal corresponding to the processing parameters and arrival of the processing target data are asynchronous with each other can be omitted.

(Second Embodiment) Packet division/restoration

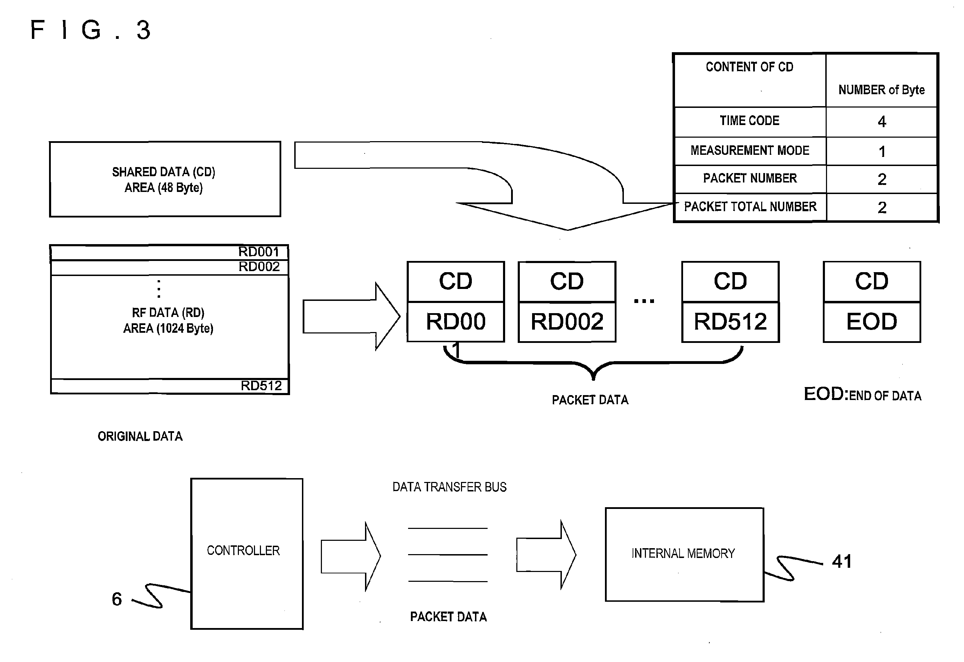

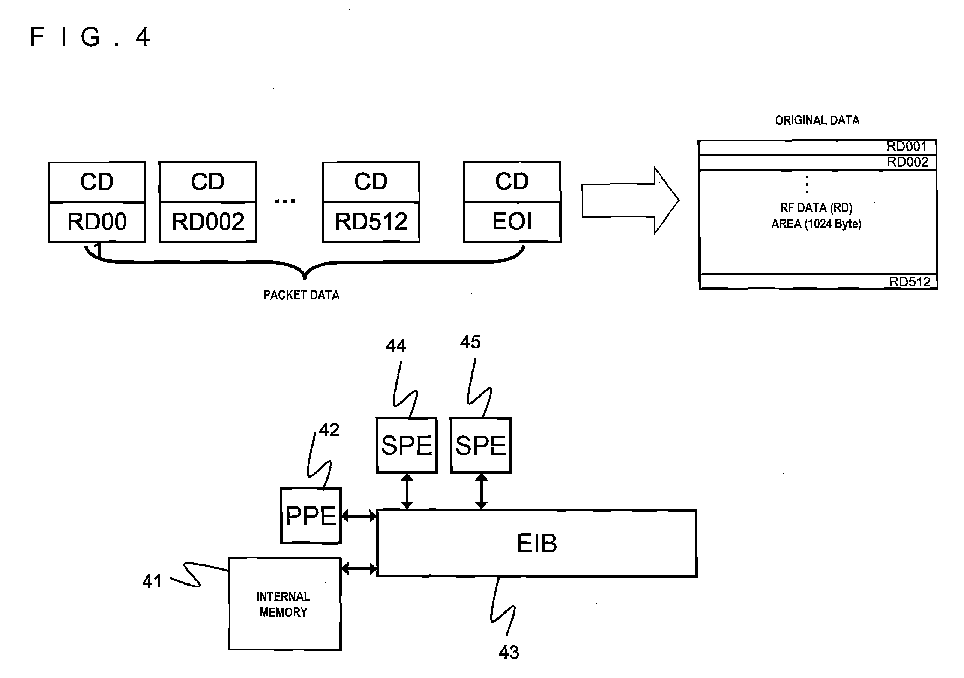

A second embodiment will be described with reference to Figs. 3 and 4. It is disclosed in the second embodiment that for example when plural measurement modes such as the B mode and the D mode are alternately measured, reduction of a frame rate in each measurement mode is prevented.

Fig.3 is a diagram showing a construction of generating packet data from original data and a data flow thereof, and Fig. 4 is a diagram showing a construction of restoring original data from packet data and a data flow thereof.

As in the case of the first embodiment, there is assumed occurrence of a situation that when the processing target data (RD) ("original data" as alias name) is transmitted with keeping 1024 bytes, the data transmission bus for connecting the controller (CONT) 6 and the data converter (DSC) 4 cannot transmit next measurement mode data until transmission is finished. For example, a standby state of measurement is expected when the B mode and the D mode are alternately measured or the like. In order to avoid this sate, a packet communication technique of communicating measurement data while dividing the measurement data is used.

In order to create the packet data, it is necessary to add the shared data (CD) with each data of a time code representing a measurement time, a packet number and the total number of packets in the measurement mode of the first embodiment and also perform data division of the processing target data (RD). When the shared data (CD) is added with the respective data described above and preliminary data are contained, it is equal to about 48 bytes, for example.

With respect to the specification of the ultrasonic diagnostic apparatus, the scan processing of ultrasonic signals to be successively measured is required to be performed on a real-time basis. The dividing number is determined in accordance with this requirement.

Furthermore, high functionalization such as increase of the number of channels of the ultrasonic probe, a situation that data processing having plural measurement modes is executed in parallel, shift of transmission/reception repeat frequency of ultrasonic waves from 8kHz to high frequency of about 20kHz, etc. are considered. For example, it is expected to require high-speed communication of about 20 times of 1 (shared data (CD)) + 1024 (processing target data (RD)) = 1025 bytes in the first embodiment.

Accordingly, 1025 bytes $\div$ 20 $\approx$ 51.2 bytes

$$51.2 - 48 = 3.2 \text{ bytes}$$

Accordingly, with respect to the number of transmissible bytes, every 2 bytes smaller than 3.2 bytes, that is, 2 + 48 = 50 bytes may be set to the size of the packet data, and the dividing number of the processing target data (RD) may be set to 512.

The controller 6 has the internal memory 61 as in the case of the first embodiment, and thus the description thereof is omitted.

The internal memory 61 stores the shared data (CD) containing the measurement mode data. The shared data (CD) has a storage area of 48 bytes as an example.

The internal memory 61 stores data (RD001 to RD512) obtained by dividing the measured original data into 512 parts. The divided data have a storage area of 2 bytes.

The controller 6 has a function of establishing data set for the storage area of CD and the storage area of the divided data (RD001 to RD512) from the original data in the internal memory 61, and generating packet data. The controller 6 also has a function of transmitting the generated packet data to the memory 41 of the data converter 4 (DSC). Packet data of 512 are stored with the time code: a measured time from a timer contained in the controller 6, measurement mode; B mode, packet number: 1 to 513, packet total number: 513, for example. The packet data of 512 are a data set of CD (48 bytes) and RD001 (2 bytes), a data set of shared data (CD) (48 bytes) and RD002 (2 bytes), ... a data set of CD (48 bytes) and RD512 (2 bytes) . A data end code (EOD) is set in the last packet data to communicate end of the packet data to the data converter 4.

The processor controller 42 of the data converter 4 receives packet data of 513 from the controller 6, and stores the packet data in the internal memory 41. The processor controller 42 analyzes the shared data (CD) and EOD to restore original data. As an analysis example, the time code and measurement mode of the shared data (CD) are analyzed, whereby the original data is restored from the packet data of the same measurement mode (in this case, B mode) which is obtained at the same time. Furthermore, with respect to the original data, the processor controller 42 assigns processing programs to the processors 44 to 4B, and obtains a B mode image from data stored in the internal memory 41.

The operation of the second embodiment will be described with reference to Figs. 3 and 4.

The controller 6 makes the addresses of the storage area of the shared data (CD) and the storage area of the divided data of the processing target data (RD) in the internal memory 61 consecutive to establish the data set, and generates packet data of 513.

The controller 6 transmits the generated packet data of 513 to the memory 41 of the data converter 4.

The processor controller 42 analyzes the time code and the measurement mode in the packet data of 513, thereby restoring the original data from the packet data of the same measurement mode (in this case, B mode) obtained at the same time. Furthermore, with respect to the original data, the processor controller 42 assigns the processing programs to the processors 44 to 4B, and obtains a B mode image from the data stored in the internal memory 41.

According to the second embodiment, the controller 6 generates the packet data, transmits the packet data to the memory 41 of the data converter 4, makes the data converter 4 analyze the measurement mode data in the packet data, and controls the data converter 4 so that the ultrasonic image data is converted from the echo data for which the packet data are restored on the basis of the analyzed measurement mode data. Therefore, the data operation processing speed can be increased by even one data converter. Furthermore, the effect of the second embodiment can satisfy the requirements of the speed-up and the high functionalization containing plural measurement modes and shift of the transmission/reception repeat frequency of ultrasonic waves to high frequency.

(Third Embodiment) calculation of B mode image/pipeline processing

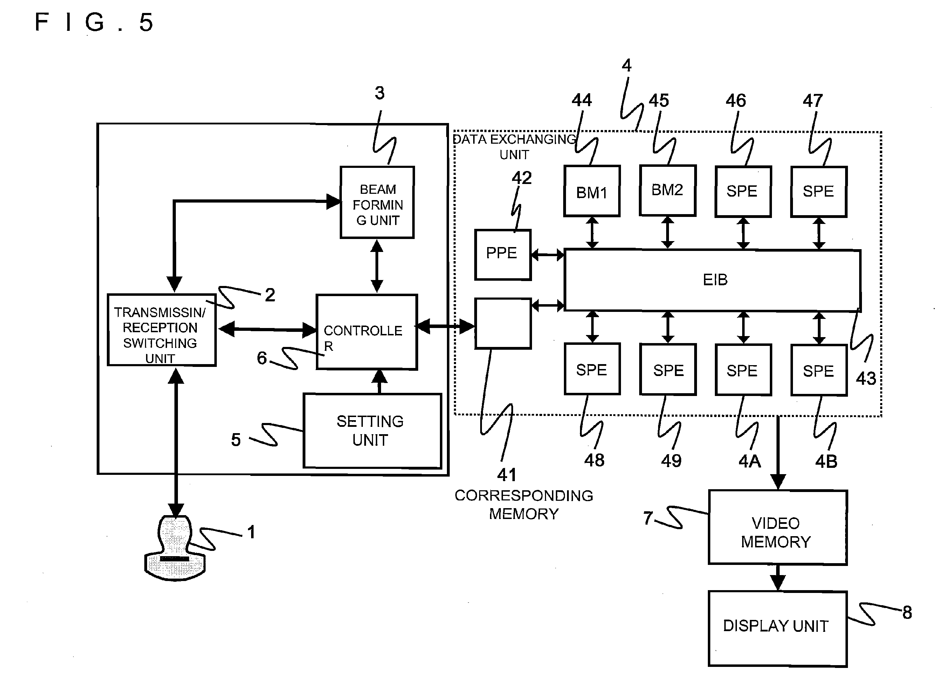

A third embodiment will be described with reference to Figs. 5, 6 and 7. In the third embodiment, an example of imaging a B mode image will be disclosed.

Fig. 5 is a block diagram showing an example in which the construction of the ultrasonic diagnostic apparatus of Fig. 1 is made to process a B mode image. In this case, in order to perform the image processing of the B mode image at high speed, a processor which executes data processing is provided to two processors of the processor 44 and the processor 45. It is assigned as BM1 and BM2 to the processors 44 and 45, respectively.

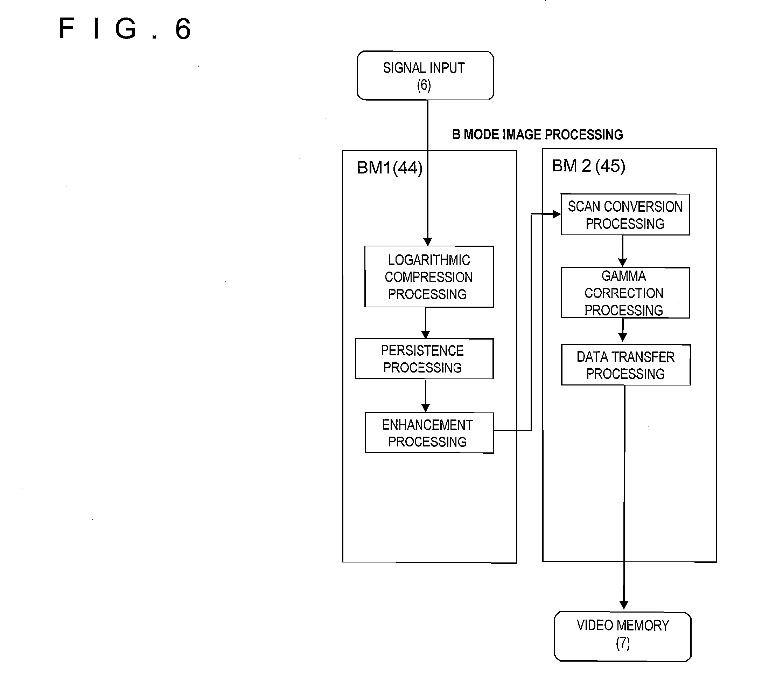

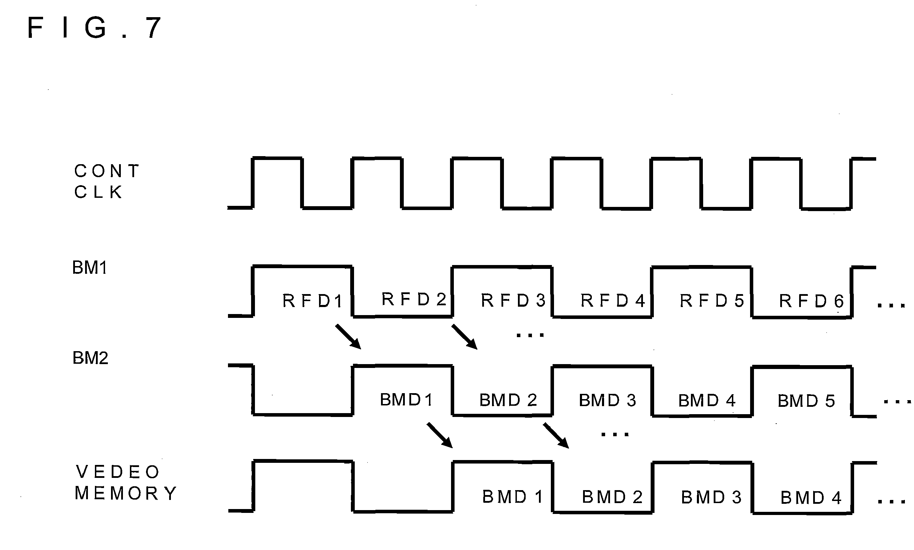

Fig. 6 is a diagram showing a processing flow in which the processing is assigned to BM1 and BM2 of Fig. 5 in order to perform specific processing with the pipeline system until a B mode image is generated, and Fig. 7 is a time chart showing an executing cycle of data processing of BM1, BM2 of Fig. 5.

The processing necessary until the B mode image is generated roughly includes logarithmic compression processing, persistence processing, enhancement processing, scan conversion processing, gamma correction processing and data transfer processing.

The dynamic range of the ultrasonic reception signal which is equal to twentieth power of 2 is compressed to a dynamic range on a relatively small circuit, substantially the dynamic range of a monitor by the logarithmic compression processing. The persistence processing executes sum and average on data displayed at the same position on the monitor for pixels after the logarithmic compression processing. The enhancement processing executes edge reinforcement on the pixels after the persistence processing so that the boundary between pixels is sharp. The scan conversion processing subjects the pixels after the enhancement processing to coordinate conversion from ultrasonic beam scanning to display monitor scanning. The gamma correction processing corrects the display gradation by a gamma curve for determining the domain and the range for pixels for the pixels after the scan conversion processing. The data transfer processing transmits an image after the gamma correction processing (B mode image) to the video memory 7.

These processing can be classified into ultrasonic scan processing including the logarithmic compression processing, the persistence processing and the enhancement processing, and TV scan processing including the scan conversion processing, the gamma correction processing and the data transfer processing. That is, the ultrasonic scan processing is assigned to BM1, and the TV scan processing is assigned to BM2. Accordingly, the pipeline system can be adopted for the data gamma correction processing of the B mode image which is successively updated.

Next, the operation of the data processing will be described with reference to Fig. 7

First, the processor 44 (BM1) executes the ultrasonic scan processing on RF1 which is first processing target data (RD).

Next, the processor 45 (BM2) executes the TV scan processing on RF1, and outputs the B mode image data (BMDI) corresponding to RF1 to the video memory 7. The processor 44 (BM1) executes the ultrasonic scan processing on RF2 as data next to RF1 in the same pipeline operation executing cycle.

Furthermore, the video memory 7 displays BMD1 on the display 8. In the same pipeline operation executing cycle, the processor 45 (BM2) executes the TV scan processing on RF2, and outputs the B mode image data (BMD2) corresponding to RF2 to the video memory 7. Furthermore, in the same pipeline operation executing cycle, the processor 44 (BM1) executes the ultrasonic scan processing on RF3 as data next to RF2.

Furthermore, the video memory 7 subsequently displays BMD2 on the display 8. In the same pipeline operation executing cycle, the processor 45 (BM2) executes the TV scan processing on RF3, and outputs the B mode image data (BMD3) for RF3 to the video memory 7. In the same pipeline operation executing cycle, the processor 44 (BM1) executes the ultrasonic scan processing on RF4 as data next to RF3.

Subsequently, the operation of the pipeline system constructed in three stages as described above is repeated until B mode image data (BMDn) for RFn (n represents a natural number) is displayed on the display 7.

In the third embodiment, the data converter 4 includes the plural processor units 44 to 4B, and the controller 6 assigns at least one of the processor units 44 to 4B to the operation of the ultrasonic image data of the measurement mode, and controls the assigned processor unit so as to perform the conversion from the echo data to the ultrasonic image data.

Furthermore, when the ultrasonic image data of the measurement mode is operated by using plural assigned processor units, the controller 6 controls the assigned processor units so as to execute the pipeline processing on the processor units at the plural stages.

Accordingly, the third embodiment has the construction described above, and thus the data operation processing can be speeded up even by one data converter. Furthermore, the effect of the third embodiment can satisfy the requirement in the high speed and high functionalization by the parallel data processing or the pipeline processing.

(Fourth Embodiment) When dedicated processor is provided for demultiplexer (DEMUX) processing

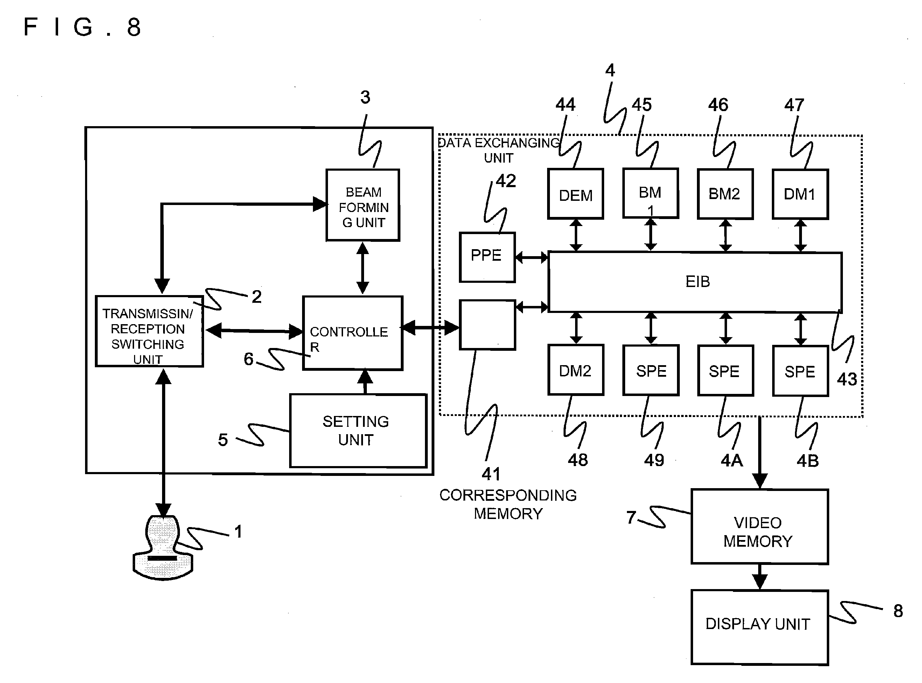

A fourth embodiment will be described with reference to Figs. 8, 9 and 10. The fourth embodiment discloses an example in which when a B mode image and a D mode measurement are alternately measured, DEMUX processing for sorting the data of the respective modes is assigned to one processor, and every two processors are assigned to each of B mode image and D mode measurement so that the B mode image and the D mode measurement are imaged.

Fig. 8 is a block diagram showing an example in which the construction of the ultrasonic diagnostic apparatus of Fig. 1 is made to perform the DEMUX processing, the B mode, D mode measurement. In this case, the processor 44 is assigned to the DEMUX processing, the processor for executing the data processing on the B mode image is assigned to the two processors of the processor 45 and the processor 46, and the processor for executing the data processing on the D mode measurement is assigned to the two processors of the processor 47 and the processor 48. These processors 44 to 48 are assigned as DEM, BM1, BM2, DM1, DM2.

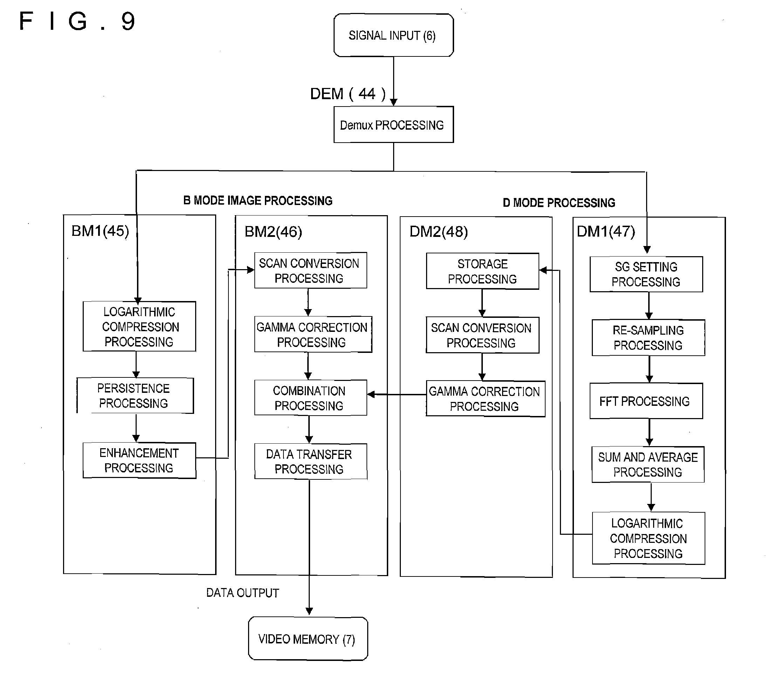

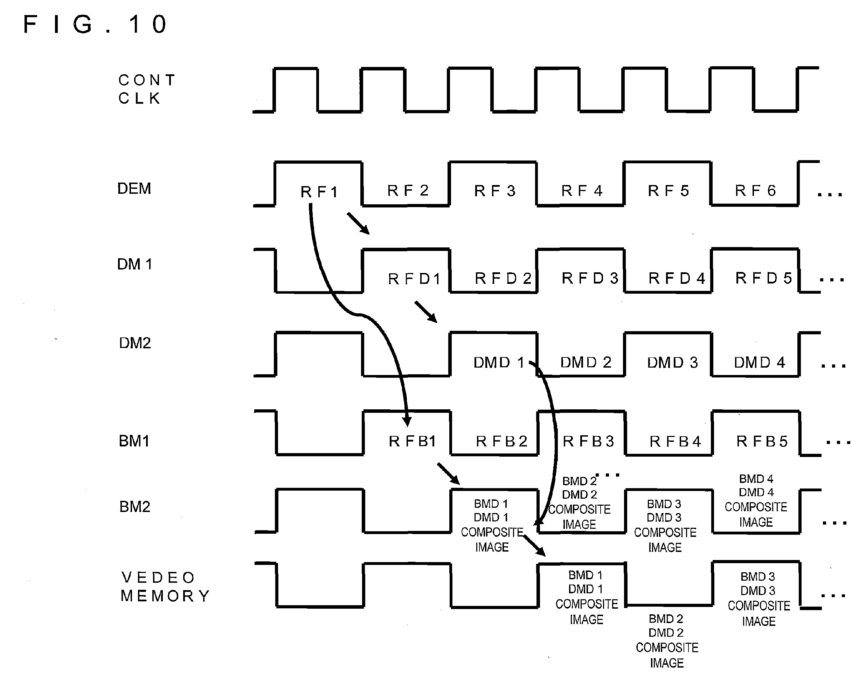

Fig. 9 is a diagram showing a processing flow in which processing is assigned to DEM, BM1, BM2, DM1, DM2 of Fig. 8 to perform the specific processing to the imaging of the B mode image and the D mode measurement by the pipeline system, and Fig. 10 is a time chart showing the executing cycle of data processing of DEM, BM1, BM2, DM1, DM2 of Fig. 8.

The DEMUX processing determines which measurement mode the processing target data corresponds to, and shares the processing target data to a B mode processor and a D mode processor on the basis of this determination.

In the fourth embodiment, the processor 44 (DEM) shares the processing target based on the measurement mode. The processor 44 (DEM) reads out data from the measurement mode in the data set in the first embodiment and in the packet data in the second embodiment, and shares the processing target data to the processor 45 (BM1) for the B mode or to the processor 47 (DM1) for the D mode on the basis of the read-out measurement mode data.

With respect to the processing required until the B mode image is generated, a part thereof described with reference to the third embodiment is omitted from the description, and only a different part will be described. The TV scan processing is added with combination processing of combining a measurement result of the D mode and the B mode image which is described later as processing to be inserted between the gamma correction processing and the data transfer processing. Furthermore, the data transfer processing transmits the image after the combination processing (the composite image of the B mode image and the D mode measurement) to the video memory 7.

The processing required until the D mode measurement roughly includes sample gate (SG) setting processing, re-sampling processing, Fast Fourier Transform (FFT) processing, sum and average processing, logarithmic compression processing, storage processing, scan conversion processing and gamma correction processing.

The SG setting processing set SG at a desired blood flow rate diagnosing site on an ultrasonic B mode image of the examinee. The re-sampling processing interpolatively determines a sample point to be subjected to Fourier Transform at a rear stage. The FFT processing executes frequency analysis on a sample point interpolated by the re-sampling processing, and removes a relatively low-frequency clutter component from a reflection body of a low moving speed such as cardiac muscle or the like to extract a blood flow component of a relatively high frequency. The sum and average processing obtains a so-called correlation value of each sample point for the blood flow component extracted by the FFT processing. The logarithmic compression processing substantially compresses the dynamic range of the sum and average processing result to the dynamic range of the monitor. The storage processing stores a logarithmic compression processing result into the internal memory 41. The scan conversion processing executes the coordinate conversion from the scan of the ultrasonic beam to the scan of the display monitor on the pixels stored in the internal memory 41. The gamma correction processing corrects the display gradation by a gamma curve for determining the domain and range of pixels for the pixels after the scan conversion processing, and outputs the correction result to the composite processing.

These D mode measurement processing can be classified into ultrasonic scan processing including the SG setting processing, the re-sampling processing, the Fast Fourier Transform (FFT) processing, the sum and average processing and the logarithmic compression processing, and TV scan processing including the gamma correction processing. That is, the ultrasonic scan processing is assigned to DM1, and the TV scanning processing is assigned to DM2. Accordingly, the pipeline system can be adopted for the data processing of the D mode measurement which is successively updated.

Next, the operation of the data processing will be described with reference to Fig. 10.

First, the processor 44 (DEM) executes data sharing processing on RF1 as first processing target data (RD). In this case, since data processing of the B mode and data processing of the D mode progress in parallel to the clock (CONT CLK) of the controller 6, the measurement data RFD1 of the D mode and the measurement data RFB1 of the B mode are contained in RF1. In this sense, the measurement data RFD2, ..., RFDn of the D mode and the measurement data RFB2, ..., RFBn of the B mode are contained in RF2, ..., RFn (n represents a natural number). The processor 44 (DEM) shares RFD1 to the processor 47 (DM1) and shares RFB1 to the processor 45 (BM1).

Subsequently, the processor 45 (BM1) executes the ultrasonic scan processing (B mode) on RFB1. In the same pipeline operation executing cycle, the processor 47 (DM1) executes the ultrasonic scan processing (D mode) on RFD1. In the same pipeline operation executing cycle, the processor 44 (DEM) shares RFD2 to the processor 47 (DM1) and shares RFB2 to the processor 45 (BM1).

Furthermore, subsequently, the processor 48 (DM2) executes the TV scan processing (D mode) on RFD1 to generate D mode measurement data (DMD1) for RFD1. The processor 46 (BM2) executes the TV scan processing (B mode) on RFB1 to generate B mode image data (BMD1) for RFB1, and combines the B mode image data (BMD1) with the D mode measurement data (DMD1). In the same pipeline operation executing cycle, the processor 46 (BM2) executes the ultrasonic scan processing (B mode) on RFB2. In the same pipeline operation executing cycle, the processor 48 (DM2) executes the ultrasonic scan processing (D mode) on RFD2. In the same pipeline operation executing cycle, the processor 44 (DEM) shares RFD3 to the processor 47 (DM1) and shares RFB3 to the processor 45 (BM1).

Subsequently, the processor 48 (DM2) executes the TV scan processing (D mode) on RFD2 to generate D mode measurement data (DMD2) for RFD2. The processor 46 (BM2) executes the TV scan processing on RFB1 to generate B mode image data (BMD2) for RFB2 and combines the B mode image data (BMD2) with the D mode measurement data (DMD2). The processor 45 (BM1) executes the ultrasonic scan processing (B mode) on RFB2. In the same pipeline operation executing cycle, the processor 47 (DM3) executes the ultrasonic scan processing (D mode) on RFD2. In the same pipeline operation executing cycle, the processor 44 (DEM) shares RFD4 to the processor 47 (DM1), and shares RFB4 to the processor 45 (BM1).

Further subsequently, the pipeline processing constructed by five stages is repeated until B mode image data (BMDn) for RFn (n represents a natural number) is displayed on the display 7.

In the fourth embodiment, the data converter 4 includes the plural processor units 44 to 4B, and the controller 6 assigns at least one of the processor units 44 to 4B to the operation of the ultrasonic image data of the measurement data, and controls the assigned processor unit so as to perform the conversion from the echo data to the ultrasonic image data.

Furthermore, when plural stages of assigned processor units are used to operate the ultrasonic image data of the measurement mode, the controller 6 controls the assigned processor units so as to execute the pipeline processing on the plural stages of the processor units.

Accordingly, when the controller 6 controls the plural processor units (44 to 4B) to advance plural measurement modes, in order to distribute the data set of each individual measurement mode to any processor unit to perform the plural measurement modes, a processor unit 44 different from the any processor unit is assigned, and the processor unit 44 different from the any processor unit and the any processor unit are controlled, so that the data operation processing speed can be increased by even one data converter. An effect inherent to the fourth embodiment resides in that the access efficiency to the internal memory 41 can be increased.

(Fifth Embodiment) When demultiplexer (DEMUX) processing is dispersed to processors

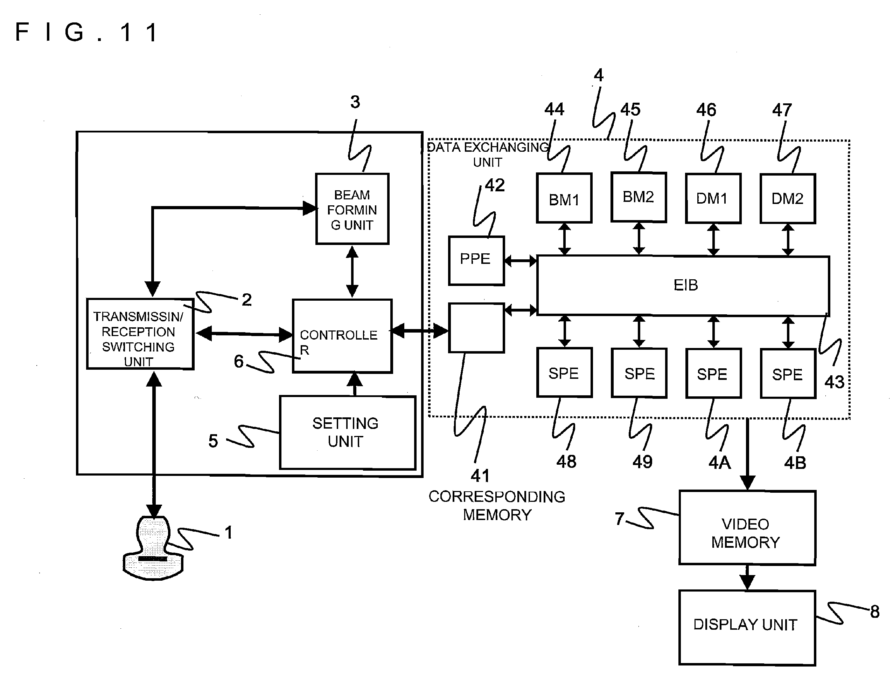

A fifth embodiment will be described with reference to Figs. 11, 12 and 13. The fifth embodiment discloses an example in which when the B mode image and the D mode measurement are alternately measured, every two processors are assigned to each of the B mode image and the D mode measurement in parallel to the DEMUX processing of sorting the data of each mode, and the B mode image and the D mode measurement are imaged by each processor.

Fig. 11 is a block diagram showing an example in which the construction of the ultrasonic diagnostic apparatus of Fig. 1 is made to process the B mode (containing the DEMUX processing) and the D mode measurement (containing the DEMUX processing). In this case, the processor for executing the DEMUX processing on the B mode image and executing the data processing on the B mode image is assigned to two processors of the processor 44 and the processor 45, and the processor for executing the DEMUX processing on the D mode image and executing the data processing on the D mode measurement is assigned to two processors of the processor 46 and the processor 47. The assigned processors 44 to 47 are represented by BM1, BM2, DM1, DM2, respectively.

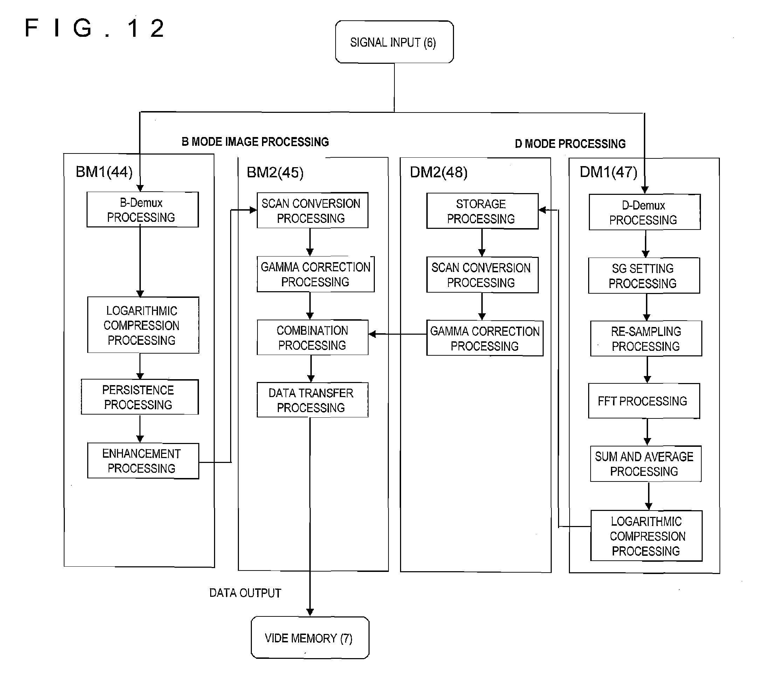

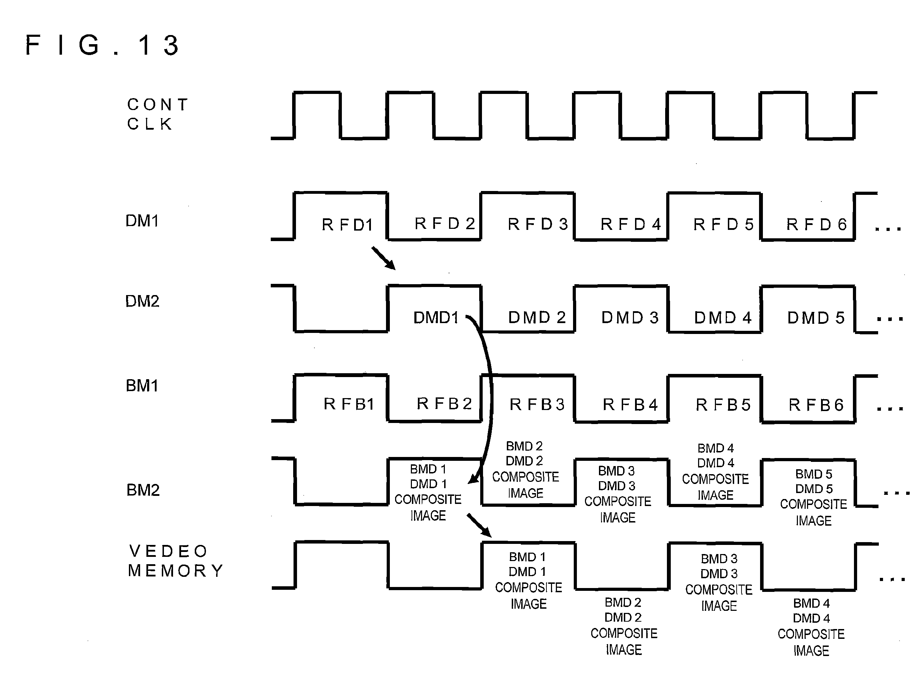

Fig. 12 is a diagram showing a processing flow in which the processing is assigned to BM1, BM2, DM1, DM2 of Fig. 11 in order to perform the specific processing to the imaging of the B mode image and the D mode measurement by the pipeline system, and Fig. 13 is a time chart representing an execution cycle of data processing of BM1, BM2, DM1, DM2 of Fig. 11.

The D-DEMUX processing determines whether the processing target data is a D mode or not, and takes the processing target data into the processor 46 on the basis of this determination. The B-DEMUX processing determines whether the processing target data is a B mode, and takes the processing target data into the processor 44 on the basis of this determination.

In the fifth embodiment, the processor 44 (BM1) takes the processing target based on this measurement mode. The processor 44 (BM1) reads data from the measurement mode in the data set in the first embodiment or in the packet data in the second embodiment, and takes the processing target data into the processor 45 (BM1) on the basis of the read-out measurement mode data in the case of the B mode. The processor 46 (DM1) takes the processing target based on this measurement mode. The processor 46 (DM1) reads out data from the measurement mode in the data set in the first embodiment or in the packet data in the second embodiment, and takes the processing target data into the processor 46 (DM1) on the basis of the read-out measurement mode data in the case of the D mode.

With respect to the processing required until generation of the B mode image and the processing required until the D mode measurement, the portion described with reference to the fourth embodiment is omitted from the description.

The processing required till the generation of the B mode image roughly includes the B-DEMUX processing, the logarithmic compression processing, the persistence processing, the enhancement processing, the scan conversion processing, the gamma correction processing and the data transfer processing.
The B-DEMUX processing identifies processing data in which the measurement mode is only the B mode, and reads out it into the processor 44.

The D mode measurement processing roughly includes the D-DEMUX processing, the SG setting processing, the re-sampling processing, the Fast Fourier Transform (FFT) processing, the sum and average processing, the logarithmic compression processing and the gamma correction processing. The B-DEMUX processing identifies processing data in which the measurement data is only the D mode, and reads it into the processor 46.

Next, the operation of the data processing will be described with reference to Fig. 13.
First, the processor 44 (BM1) executes the data take-in processing on RF1 as first processing target data (RD), and executes the ultrasonic scan processing (B mode) on RFB1. In the same pipeline operation executing cycle, the processor 46 (DM1) executes the ultrasonic scan processing (D mode) on RFD1.

Subsequently, the processor 46 (DM2) executes the TV scan processing (D mode) on RFD1 to generates D mode measurement data (DMD1) for RFD1. The processor 44 (BM2) executes the TV scan processing on RFB1 to generate the B mode image data (BMD1) for RFB1, and combines the B mode image data (BMD1) with the D mode measurement data (DMD1) The processor 44 (BM1) executes the ultrasonic scan processing (B mode) on RFB2. In the same pipeline operation executing cycle, the processor 46 (DM2) executes the ultrasonic scan processing (D mode) on RFD2.

Further subsequently, the processor 47 (DM2) executes the TV scan processing (D mode) on RFD2 to generate the D mode measurement data (DMD2) for RFD2. The processor 45 (BM2) executes the TV scan processing on RFB1 to generate the B mode image data (BMD2) for RFB2, and combines the B mode image data (BMD2) with the D mode measurement data (DMD2). The processor 44 (BM1) executes the ultrasonic scan processing (B mode) on RFB2. In the same pipeline operation executing cycle, the processor 47 (DM3) executes the ultrasonic scan processing (D mode) on RFD2. In the same pipeline operation executing line, the processor 44 (BM1) shares RFB2 to the processor 45 (BM1).

Further subsequently, the pipeline processing constructed by four stages is repeated until B mode image data (BMDn) for RFn (n represents a natural number) is displayed on the display 7.

In the fifth embodiment, the data converter 4 includes plural processor units 44 to 4B, and the controller 6 assigns to at least one of the processor units 44 to 4B to operate the ultrasonic image data of the measurement mode, and controls the assigned processor unit so as to perform the conversion from the echo data to the ultrasonic image data.

Furthermore, when the ultrasonic image data of the measurement mode is operated by using plural stages of assigned processor units, the controller 6 may control the assigned processors so as to execute the pipeline processing on the plural stages of processor units.

Accordingly, in the fifth embodiment, when the controller 6 makes the plural processor units 44 to 4B to advance the data processing of the plural measurement mode in parallel, any processor unit which is made to perform the plural measurement modes can be made to identify the data set of an individual measurement mode by the controller 6.

Furthermore, the controller 6 can control an adjustment between a frame measurement mode of shifting an image on a frame basis out of the plural measurement modes and a survey measurement mode of shifting an image on a line basis out of the plural measurement modes.

As described above, in the fifth embodiment, the speed of the data operation processing can be increased by even one data converter. Furthermore, an effect inherent to the fifth embodiment resides in that it can be easily installed into the processor dividing processing.

(Sixth Embodiment) Stop of progression of processing when error is detected

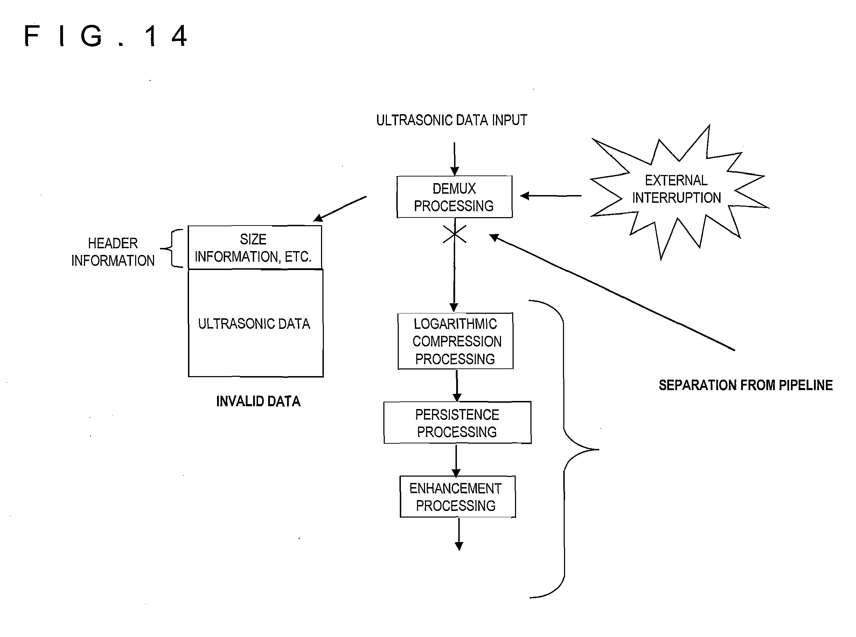

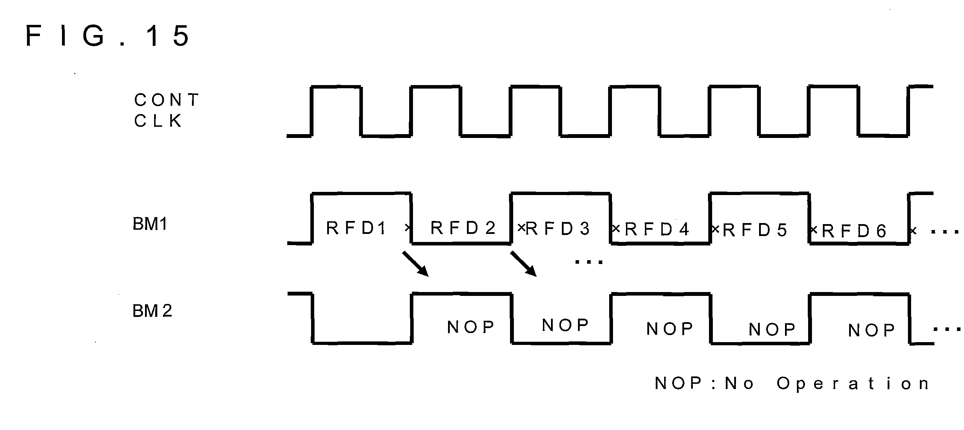

A sixth embodiment will be described with reference to Figs. 14 and 15. The sixth embodiment discloses an example in which in the measurement of the B mode image, execution of the subsequent processing after an error is detected is stopped.

Fig. 14 is a diagram showing a processing flow in which the pipeline of specific processing until imaging of the B mode image is interrupted, and Fig. 15 is a time chart showing an execution cycle of data processing of BM1 of Fig. 14.

In the sixth embodiment, the processor 44 (BM1) finds an error in shared data. That is, the processor 44 (BM1) reads out error data in the data set in the first embodiment or in the packet data in the second embodiment, and stops the operation of subsequently connected processor, video memory and display on the basis of the read-out error data, and executes NOP (No Operation) program.

In the sixth embodiment, the controller 6 further adds the data set with error data under the data transfer or the measurement, makes the data converter 4 analyze the error data in the data set, and controls the data converter 4 to stop the conversion from the echo data in the data set to the ultrasonic image data on the basis of the analyzed error data.

Accordingly, an effect inherent to the sixth embodiment resides in that the data operation of invalid data can be stopped when error information is detected.

Furthermore, the preferred embodiments of the ultrasonic diagnostic apparatus, etc. of this invention have been described with reference to the accompanying drawings, however, the present invention is not limited to these examples. It is apparent to persons skilled in the art that various alterations and modifications can be performed within the scope of the technical idea disclosed in this application, and it is understood that they belong to the technical scope of this invention.

Furthermore, in this embodiment, the controller 6 is provided in the processor. However, the present invention is not limited to this embodiment, and the controller 6 may be provided at the outside of the processor.

Description of Reference Numerals

1 ultrasonic probe, 3 beam forming unit, 4 data converter, 5 setting unit, 6 controller

## Claims

1. An ultrasonic diagnostic apparatus having an ultrasonic probe for transmitting an ultrasonic beam to an examinee and receiving a reflection echo signal from the examinee, a beam forming unit for supplying the ultrasonic probe with a signal for making the ultrasonic probe transmit an ultrasonic beam, and a data converter for digitalizing the reflection echo signal to obtain echo data, and converting the echo data to ultrasonic image data, comprising:

   a setting unit that sets measurement mode data of the ultrasonic image data in the echo data; and
   a controller that creates a data set while appending the measurement mode data to the echo data, transferring the data set to the data converter, makes the data converter analyze the measurement mode data in the data set, and controls the data converter so that echo data in the data set are converted to ultrasonic image data on the basis of the analyzed measurement mode data.

2. The ultrasonic diagnostic apparatus according to claim 1, wherein the controller generates, as the data set, packet data containing divisional data obtained by dividing the echo data into a plurality of parts and the measurement mode data.

3. The ultrasonic diagnostic apparatus according to claim 2, wherein the controller further adds the packet data with measurement time information at which the echo data is obtained, and the data converter restores the echo data from the packet data by using the measurement time information.

4. The ultrasonic diagnostic apparatus according to claim 1, wherein the data converter comprises a plurality of processor units, and the controller assigns at least one of the processor units to operate ultrasonic image data of the measurement mode, and controls the assigned processor unit to perform conversion from the echo data to ultrasonic image data.

5. The ultrasonic diagnostic apparatus according to claim 4, wherein when the controller operates the ultrasonic image data of the measurement mode by using a plurality of stages of assigned processor units, the controller controls the assigned processor units so as to perform pipeline processing on the plurality of stages of processor units.

**6.** The ultrasonic diagnostic apparatus according to claim 4, wherein when the controller makes the plurality of processor units advance a plurality of measurement modes, in order to distribute a data set of each individual measurement mode to any processor unit which is made to perform the plurality of measurement modes, the controller assigns a processor unit different from the any processor unit, and controls the processor unit different from the any processor unit and the any processor unit.

**7.** The ultrasonic diagnostic apparatus according to claim 4, wherein when the controller makes the plurality of processor units advance a plurality of measurement modes, any processor unit which is made to perform the plurality of measurement modes is made to identify a data set of each individual measurement mode by the controller.

**8.** The ultrasonic diagnostic apparatus according to claim 7, wherein the controller controls an adjustment between a frame measurement mode of shifting an image on a frame basis out of the plurality of measurement modes and a survey measurement mode of shifting an image on a line basis out of the plurality of measurement modes.

**9.** The ultrasonic diagnostic apparatus according to claim 1, wherein the controller further adds the data set with error data under data transfer or measurement, makes the data converter analyze the error data in the data set, and controls the data converter to stop the conversion from the echo data in the data set to the ultrasonic image data on the basis of the analyzed error data.

**10.** A data processing method for an ultrasonic diagnostic apparatus for transmitting an ultrasonic beam to an examinee and receiving a reflection echo signal from the examinee by an ultrasonic probe, supplying the ultrasonic probe with a signal for making the ultrasonic probe transmit an ultrasonic beam by a beam forming unit, and digitalizing the reflection echo signal to obtain echo data and converting the echo data to ultrasonic image data by a data converter, further comprising:

setting measurement mode data of the ultrasonic image data in the echo data by a setting unit; and
generating a data set while appending the measurement mode data and the echo data, transferring the data set to the data converter, making the data converter analyze measurement mode data in the data set, and controlling the data converter so as to convert echo data in the data set to ultrasonic image data on the basis of the analyzed measurement mode data by a controller.

**11.** The data processing method for the ultrasonic diagnostic apparatus according to claim 8, wherein the controller generates, as the data set, packet data containing divisional data obtained by dividing the echo data into a plurality of parts and the measurement mode data.

**12.** The data processing method for the ultrasonic diagnostic apparatus according to claim 11, wherein the controller further adds the packet data with measurement time information at which the echo data is obtained, and the data converter restores the echo data from the packet data by using the measurement time information.

**13.** The data processing method for the ultrasonic diagnostic apparatus according to claim 10, wherein the data converter comprises a plurality of processor units, and the controller assigns at least one of the processor units to operate ultrasonic image data of the measurement mode, and controls the assigned processor unit to perform conversion from the echo data to ultrasonic image data.

**14.** The data processing method for the ultrasonic diagnostic apparatus according to claim 11, wherein the controller further adds the data set with error data under data transfer or measurement, makes the data converter analyze the error data in the data set, and controls the data converter to stop the conversion from the echo data in the data set to the ultrasonic image data on the basis of the analyzed error data.

F I G . 1

DATA EXCHANGING UNIT

BEAM FORMING UNIT

3

TRANSMISSIN/ RECEPTION SWITCHING UNIT

2

CONTROLLER

6

SETTING UNIT

5

PPE

42

SPE   44

SPE   45

SPE   46

SPE   47

EIB

43

SPE   48

SPE   49

SPE   4A

SPE   4B

4

CORRESPONDING MEMORY

41

1

VIDEO MEMORY

7

DISPLAY UNIT

8

EP 2 324 769 A1

# F I G . 2 A

SHARED DATA (CD)
AREA (1 Byte)

RF DATA (RD)
AREA (1024 Byte)

61

6

ORIGINAL DATA

| CONTENT OF CD | NUMBER OF Byte |
|---|---|
| MEASUREMENT MODE | 1 |

# FIG. 2B

CONTROLLER

6

DATA TRANSFER BUS

ORIGINAL DATA

INTERNAL MEMORY

41

EP 2 324 769 A1

# F I G . 3

| CONTENT OF CD | NUMBER of Byte |
|---|---|
| TIME CODE | 4 |
| MEASUREMENT MODE | 1 |
| PACKET NUMBER | 2 |
| PACKET TOTAL NUMBER | 2 |

SHARED DATA (CD)
AREA (48 Byte)

| RD001 |
| RD002 |
| ⋮ |
| RF DATA (RD)
AREA (1024 Byte) |
| RD512 |

ORIGINAL DATA

| CD | | CD | | | CD | | CD |
|---|---|---|---|---|---|---|---|
| RD00 | | RD002 | ... | | RD512 | | EOD |

PACKET DATA

EOD:END OF DATA

DATA TRANSFER BUS

CONTROLLER

6

PACKET DATA

INTERNAL MEMORY

41

EP 2 324 769 A1

ORIGINAL DATA

| CD | | CD | | CD | | CD |
|----|---|----|---|----|---|----|
| RD00 | | RD002 | ... | RD512 | | EOI |

PACKET DATA

| | RD001 |
|---|---|
| | RD002 |
| RF DATA (RD) AREA (1024 Byte) | ⋮ |
| | RD512 |

INTERNAL MEMORY

PPE

SPE  SPE

EIB

41  42  44  45  43

EP 2 324 769 A1

# F I G . 5

FIG. 5

# F I G . 6

SIGNAL INPUT (6)

B MODE IMAGE PROCESSING

BM1(44)

LOGARITHMIC COMPRESSION PROCESSING

PERSISTENCE PROCESSING

ENHANCEMENT PROCESSING

BM 2 (45)

SCAN CONVERSION PROCESSING

GAMMA CORRECTION PROCESSING

DATA TRANSFER PROCESSING

VIDEO MEMORY (7)

EP 2 324 769 A1

# FIG.7

CONT CLK

BM1

RFD1 RFD2 RFD3 RFD4 RFD5 RFD6 ...

BM2

BMD1 BMD2 BMD3 BMD4 BMD5 ...

VEDEO MEMORY

BMD1 BMD2 BMD3 BMD4 ...

EP 2 324 769 A1

FIG.8

EP 2 324 769 A1

# F I G . 9

SIGNAL INPUT (6)

DEM ( 44 )

Demux PROCESSING

**B MODE IMAGE PROCESSING**

BM1(45)
- LOGARITHMIC COMPRESSION PROCESSING
- PERSISTENCE PROCESSING
- ENHANCEMENT PROCESSING

BM2(46)
- SCAN CONVERSION PROCESSING
- GAMMA CORRECTION PROCESSING
- COMBINATION PROCESSING
- DATA TRANSFER PROCESSING

**D MODE PROCESSING**

DM2(48)
- STORAGE PROCESSING
- SCAN CONVERSION PROCESSING
- GAMMA CORRECTION PROCESSING

DM1(47)
- SG SETTING PROCESSING
- RE-SAMPLING PROCESSING
- FFT PROCESSING
- SUM AND AVERAGE PROCESSING
- LOGARITHMIC COMPRESSION PROCESSING

DATA OUTPUT

VIDEO MEMORY (7)

EP 2 324 769 A1

FIG.10

CONT CLK

DEM  RF1  RF2  RF3  RF4  RF5  RF6  ...

DM1  RFD1  RFD2  RFD3  RFD4  RFD5  ...

DM2  DMD1  DMD2  DMD3  DMD4  ...

BM1  RFB1  RFB2  RFB3  RFB4  RFB5  ...

BM2  BMD1 DMD1 COMPOSITE IMAGE  BMD2 DMD2 COMPOSITE IMAGE  BMD3 DMD3 COMPOSITE IMAGE  BMD4 DMD4 COMPOSITE IMAGE  ...

VEDEO MEMORY  BMD1 DMD1 COMPOSITE IMAGE  BMD2 DMD2 COMPOSITE IMAGE  BMD3 DMD3 COMPOSITE IMAGE  ...

EP 2 324 769 A1

FIG. 11

EP 2 324 769 A1

# FIG. 12

SIGNAL INPUT (6)

**B MODE IMAGE PROCESSING**

**D MODE PROCESSING**

## BM1(44)

- B-Demux PROCESSING
- LOGARITHMIC COMPRESSION PROCESSING
- PERSISTENCE PROCESSING
- ENHANCEMENT PROCESSING

## BM2(45)

- SCAN CONVERSION PROCESSING
- GAMMA CORRECTION PROCESSING
- COMBINATION PROCESSING
- DATA TRANSFER PROCESSING

## DM2(48)

- STORAGE PROCESSING
- SCAN CONVERSION PROCESSING
- GAMMA CORRECTION PROCESSING

## DM1(47)

- D-Demux PROCESSING
- SG SETTING PROCESSING
- RE-SAMPLING PROCESSING
- FFT PROCESSING
- SUM AND AVERAGE PROCESSING
- LOGARITHMIC COMPRESSION PROCESSING

DATA OUTPUT

VIDE MEMORY (7)

EP 2 324 769 A1

# F I G . 1 3

CONT CLK

DM1: RFD1 RFD2 RFD3 RFD4 RFD5 RFD6 ...

DM2: DMD1 DMD2 DMD3 DMD4 DMD5 ...

BM1: RFB1 RFB2 RFB3 RFB4 RFB5 RFB6 ...

BM2:
BMD 1 DMD 1 COMPOSITE IMAGE
BMD 2 DMD 2 COMPOSITE IMAGE
BMD 3 DMD 3 COMPOSITE IMAGE
BMD 4 DMD 4 COMPOSITE IMAGE
BMD 5 DMD 5 COMPOSITE IMAGE ...

VEDEO MEMORY:
BMD 1 DMD 1 COMPOSITE IMAGE
BMD 2 DMD 2 COMPOSITE IMAGE
BMD 3 DMD 3 COMPOSITE IMAGE
BMD 4 DMD 4 COMPOSITE IMAGE ...

EP 2 324 769 A1

# F I G . 1 4

ULTRASONIC DATA INPUT

```
                    ┌──────────────┐        ┌─────────────┐
                    │    DEMUX     │◄────── │  EXTERNAL   │
                    │  PROCESSING  │        │ INTERRUPTION│
                    └──────────────┘        └─────────────┘
                           ✕
```

HEADER INFORMATION

| SIZE INFORMATION, ETC. |
|---|
| ULTRASONIC DATA |

**INVALID DATA**

```
┌───────────────┐
│  LOGARITHMIC  │
│  COMPRESSION  │
│  PROCESSING   │
└───────────────┘
        │
        ▼
┌───────────────┐
│  PERSISTENCE  │
│  PROCESSING   │
└───────────────┘
        │
        ▼
┌───────────────┐
│  ENHANCEMENT  │
│  PROCESSING   │
└───────────────┘
```

**SEPARATION FROM PIPELINE**

# FIG. 15

CONT
CLK

BM1  RFD1 × RFD2 × RFD3 × RFD4 × RFD5 × RFD6 × ...

... 

BM2  NOP  NOP  NOP  NOP  NOP  ...

NOP:No Operation

EP 2 324 769 A1

## INTERNATIONAL SEARCH REPORT

| | International application No. |
|---|---|
| | PCT/JP2009/064576 |

### A. CLASSIFICATION OF SUBJECT MATTER
A61B8/00(2006.01)i

According to International Patent Classification (IPC) or to both national classification and IPC

### B. FIELDS SEARCHED

Minimum documentation searched (classification system followed by classification symbols)
A61B8/00

Documentation searched other than minimum documentation to the extent that such documents are included in the fields searched

| | | | |
|---|---|---|---|
| Jitsuyo Shinan Koho | 1922–1996 | Jitsuyo Shinan Toroku Koho | 1996–2009 |
| Kokai Jitsuyo Shinan Koho | 1971–2009 | Toroku Jitsuyo Shinan Koho | 1994–2009 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X<br><br>Y | JP 2004-41273 A (Aloka Co., Ltd.),<br>12 February, 2004 (12.02.04),<br>Par. Nos. [0018] to [0021], [0025] to [0029];<br>Figs. 1 to 3<br>(Family: none) | 1-4,6-8,<br>10-13<br>9,14 |
| X<br><br>Y | JP 2004-351083 A (Aloka Co., Ltd.),<br>16 December, 2004 (16.12.04),<br>Par. Nos. [0025] to [0031], [0035], [0040],<br>[0048] to [0050]; Figs. 1, 3, 7, 8<br>(Family: none) | 1,2,4,5,10,<br>11<br>9,14 |
| Y | JP 2006-136711 A (General Electric Co.),<br>01 June, 2006 (01.06.06),<br>Par. No. [0053]; Fig. 4<br>& US 2006/0092930 A1 & DE 102005051353 A | 9,14 |

| ☒ Further documents are listed in the continuation of Box C. | ☐ See patent family annex. |
|---|---|

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search<br>30 September, 2009 (30.09.09) | Date of mailing of the international search report<br>13 October, 2009 (13.10.09) |
|---|---|
| Name and mailing address of the ISA/<br>Japanese Patent Office | Authorized officer |
| Facsimile No. | Telephone No. |

Form PCT/ISA/210 (second sheet) (April 2007)

**INTERNATIONAL SEARCH REPORT**

| International application No. |
| --- |
| PCT/JP2009/064576 |

C (Continuation).    DOCUMENTS CONSIDERED TO BE RELEVANT

| Category* | Citation of document, with indication, where appropriate, of the relevant passages | Relevant to claim No. |
| --- | --- | --- |
| X | JP 11-164831 A  (Aloka Co., Ltd.),<br>22 June, 1999 (22.06.99),<br>Par. Nos. [0012], [0013], [0016] to [0019];<br>Figs. 1, 2<br>(Family: none) | 1 |

Form PCT/ISA/210 (continuation of second sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3612358 B